# FASCICULE DE BREVET EUROPEEN

(11) **EP 1 913 951 B1**
(45) Date de publication et mention de la délivrance du brevet: **03.04.2019**
(21) Numéro de dépôt: 06291611.9
(22) Date de dépôt: 17.10.2006
(51) Int. Cl.: A61K 36/45, A61K 31/35, A61P 13/00, A61P 31/04, A23L 2/78

(54) **Procédé d'extraction de substances polyphénoliques et leurs utilisations**
Verfahren zur Extraktion von Polyphenolen und ihre Verwendung
Method for the extraction of polyphenols and uses thereof

(43) Date de publication de la demande: 23.04.2008
(73) Titulaire: NEXIRA, 76000 Rouen (FR)
(72) Inventeur: Renard, Loic, 92500 Rueil Malmaison (FR)
(74) Mandataire: Jouannic, Nathalie

(56) Documents cités:
- WO-A-99/12541
- US-A1- 2002 028 260
- US-A1- 2003 091 660
- US-B1- 6 676 978
- CHEN H ET AL: "Separation and determination of flavonoids and other phenolic compounds in cranberry juice by high-performance liquid chromatography" JOURNAL OF CHROMATOGRAPHY, ELSEVIER SCIENCE PUBLISHERS B.V. AMSTERDAM, NL, vol. 913, no. 1-2, 13 avril 2001 (2001-04-13), pages 387-395, XP004232805 ISSN: 0021-9673
- ZIRK M M ET AL: "Cranberry (Vaccinium Macrocarpon) proanthocyanidins and their effects on urinary tract infections" CURRENT TOPICS IN NUTRACEUTICAL RESEARCH 2004 UNITED STATES, vol. 2, no. 3, 2004, pages 153-160, XP008076009 ISSN: 1540-7535
- FOO LAI YEAP ET AL: "The structure of cranberry proanthocyanidins which inhibit adherence of uropathogenic P-fimbriated Escherichia coli in vitro" PHYTOCHEMISTRY (OXFORD), vol. 54, no. 2, mai 2000 (2000-05), pages 173-181, XP002423207 ISSN: 0031-9422
- FOO L Y ET AL: "A-type proanthocyanidin trimers from cranberry that inhibit adherence of uropathogenic P-fimbriated Escherichia coli" JOURNAL OF NATURAL PRODUCTS 2000 UNITED STATES, vol. 63, no. 9, 2000, pages 1225-1228, XP002423208 ISSN: 0163-3864
- KONTIOKARI ET AL: "Cranberry juice and bacterial colonization in children-A placebo-controlled randomized trial" CLINICAL NUTRITION, CHURCHILL LIVINGSTONE, LONDON, GB, vol. 24, no. 6, décembre 2005 (2005-12), pages 1065-1072, XP005177048 ISSN: 0261-5614
- ZHANG ZHE ET AL: "Comparison of HPLC methods for determination of anthocyanins and anthocyanidins in bilberry extracts." JOURNAL OF AGRICULTURAL AND FOOD CHEMISTRY 25 FEB 2004, vol. 52, no. 4, 25 février 2004 (2004-02-25), pages 688-691, XP002422933 ISSN: 0021-8561
- LEE JUNGMIN ET AL: "Comparison of anthocyanin pigment and other phenolic compounds of Vaccinium membranaceum and Vaccinium ovatum native to the Pacific Northwest of North America." JOURNAL OF AGRICULTURAL AND FOOD CHEMISTRY 17 NOV 2004, vol. 52, no. 23, 17 novembre 2004 (2004-11-17), pages 7039-7044, XP002422934 ISSN: 0021-8561
- DATABASE WPI Week 200446 Thomson Scientific, London, GB; AN 2004-482741 & JP 3 533392 B1 (TOYO SHINYAKU KK) 31 May 2004 (2004-05-31)
- DATABASE WPI Week 200467 Thomson Scientific, London, GB; AN 2004-690576 & WO 2004/080994 A1 (TOYO SHINYAKU CO LTD) 23 September 2004 (2004-09-23)

## Description

La présente invention se rapporte au domaine de la chimie et plus particulièrement à celui des produits bénéfiques pour la santé.

L'invention se rapporte en particulier à la chimie extractive et à l'obtention de produits naturels polyphénoliques utiles pour combattre les infections urinaires aigües ou chroniques.

Les infections urinaires sont d'une extrême fréquence. Elles viennent, après les infections respiratoires, au second rang des motifs de consultation et de prescription d'antibiotiques. Elles sont facilement récidivantes notamment chez la femme et les porteurs de vessie neurologique. L'agent microbien le plus souvent responsable de ces infections urinaires est *Escherichia coli,* bactérie commensale du tube digestif qui peut devenir pathogène par acquisition d'îlots de pathogénicité. Une autre bactérie fréquemment isolée chez les femmes jeunes non ménopausées est le staphylocoque, en particulier *S.saprophyticus* qui peut être à l'origine de pyurie. D'autres agents microbiens sont également responsables d'infections urinaires : *Proteus, Klebsiella, Enterobacter* parmi les Gram-, *Enterocoques* ou *Staphylocoques* parmi les Gram+ ainsi que des levures.

Ces infections bactériennes peuvent devenir persistantes voire même chroniques et il importe de mettre au point des médicaments efficaces mais en même temps sélectifs pour ne pas faire disparaître la flore bactérienne commensale normalement présente dans l'urètre.

L'invention a pour objet un procédé d'extraction des substances polyphénoliques et plus particulièrement des proanthocyanidines de la série A sous forme trimères à hexamères, du jus de Cranberry qui consiste à éliminer les substances inertes, par chromatographie du jus sur résine échangeuse d'ions, notamment du type styrène/divinylbenzène et en particulier par une résine Amberlite XAD 16 HP fabriquée par la société ROHM et HAAS, qui fixe les polyphénols et laisse non absorbées toutes les substances minérales ou organiques inertes présentes dans le jus de Cranberry.

Le jus de Cranberry ou Grande Airelle est tiré d'un fruit cultivé au Canada et dans le Nord des Etats-Unis. Ce jus de couleur rouge sombre et de saveur astringente est riche en polyphénols et notamment en pro anthocyanidines sous forme libre ou glycosylée (galactoside, rhamnoside, glucoside) ou estérifiée (gallate) comme par exemple la prodelphinidine, la propetunidine, la promalvidine ou la procyanidine. Ces composés existent en général sous forme polymérisée (trimères à hexamères) et possèdent un enchaînement de type A ou de type B selon les deux schémas suivants:

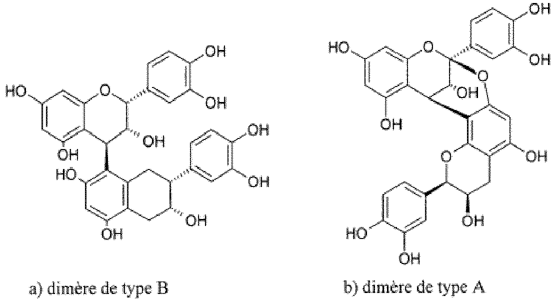

Structure d'un dimère de type B (a) comparé à un dimère (b) de type A

Le jus de Cranberry possède un taux de proanthocyanidines par rapport au produit frais, de 1,2 à 2,3 environ suivant la variété, la provenance ou le mode de culture du fruit.

L'objectif du procédé consiste donc à réaliser des concentrés de jus de Cranberry possédant un taux de proanthocyanidines d'au moins 10 %. Des jus déjà très concentrés peuvent ensuite permettre la préparation de compositions pulvérulentes titrant 60 à 90 % en polyphénols totaux.

Il est important et c'est là où se situe l'essence de l'invention de pouvoir disposer de compositions concentrées en polyphénols. Les préparations à base de Cranberry possèdent des propriétés diététiques et thérapeutiques intéressantes et notamment des propriétés anti-oxydantes et anti-adhérentes vis-à-vis des germes microbiens tels qu'Escherichia coli bactérie commensale du tube digestif. De même, on a déjà réalisé des gels ou des crèmes à forte teneur en polyphénols de Cranberry destinées à imprégner des tubes, des canules, des cathéters ou des sondes placées à demeure dans l'urètre ou la vessie.

De telles crèmes ou de tels gels sont destinés à assurer une désinfection de l'arbre urinaire en permettant l'élimination de la flore urinaire pathogène.

Les propriétés anti-adhérentes de l'extrait de Vaccinium macrocarpon sont bien connues et ont été mises à profit dans de nombreuses infections urinaires pour combattre le développement de la bactériurie. Par ailleurs, les demandeurs ont démontré, par des expériences *in vitro* (cellules urothéliales T24), que l'extrait de Cranberry avait une activité anti-adhérente significativement plus importante comparée à un placebo vis à vis de souches d'*E*. *coli* uropathogènes.

Un tel traitement pour être efficace nécessite des concentrations élevées en polyphénols et c'est pourquoi il est hautement souhaitable de disposer d'un procédé simple, facile à mettre en oeuvre, industrialisable et qui permette d'obtenir en quantités importantes des concentrés de jus de Cranberry. Les demandes de brevet WO99/12541 et US20020028260 divulguent un procédé d'extraction de proanthocyanidines à partir d'une plante, de préférence la Cranberry, dans lequel un extrait aqueux est soumis à une chromatographie en phase inverse, suivi de lavages avec de l'eau distillée puis par du méthanol aqueux à 15%, et d'une élution des polyphénols avec du méthanol acidifié à 100%. Il y est indiqué que ces proanthocyanidines sont utiles pour la prévention et le traitement des infections urinaires causées par *Escherichia coli* de type P, en inhibant ses propriétés d'agglutination et d'adhérence. Le brevet JP3533392 et la demande de brevet WO2004/080994 divulguent des procédés d'extraction de proanthocyanidines à partir d'extraits d'écorce de pin. Le brevet US6676978 se rapporte à un procédé d'extraction d'un mélange d'anthocyanidines, de bioflavonoides et de substances phénoliques à partir de jus de baies, et plus particulièrement de cerises. La demande de brevet US20030091660 décrit un procédé d'obtention d'anthocyanines à partir de jus de myrtille ou de cassis. Foo et al (Journal of Natural Products, vol. 63, no. 9, 2000, pages 1225-1228) décrit un procédé d'extraction de proanthocyanidines à partir de la Cranberry, comprenant notamment une étape d'homogénéisation des fruits avec de l'acétone et une étape de chromatographie sur colonne Sephadex LH-20, en utilisant l'éthanol comme éluant.

La présente invention a plus spécifiquement pour objet un procédé de concentration et d'isolement des fractions polyphénoliques du jus de Cranberry qui consiste en l'adsorption sur une résine du type divinyl benzène styrène non ionique, commercialisée sous la dénomination commerciale AMBERLITE® XAD 16 HP par la firme ROHM et HAAS. Il s'agit d'un polymère réticulé aliphatique macroréticulaire se présentant sous forme de billes blanches translucides retenant de l'humidité (62-70 %). Elle possède une zone de surface de ≥ 800 m²/g et une porosité de ≥ 0,55 ml/ml. Ce type de résine est neutre et convient pour n'importe quelle zone de pH. La taille des pores est d'environ 1,082 ml/g en volume et la taille des pores est d'environ 100 µm en moyenne.

Cette résine gonfle au contact de solvants organiques comme le méthanol, l'isopropanol, l'acétone ou le p-xylène.

Des produits similaires tels que la résine Amberlite XAD 4 ou Amberlite XAD 16 peuvent également être utilisés. On peut également utiliser la résine Amberlite XAD 2.

La présente invention a également pour objet un procédé d'extraction des polyphénols du type proanthocyanidines du jus de Cranberry (Vaccinium macrocarpon) qui consiste à éliminer en premier lieu les substances inertes contenues dans ce jus par dilution à l'eau du jus concentré, à fixer sur un matériau adsorbant polymère du type styrène/divinyl benzène, les polyphénols du type proanthocyanidines, à rincer la colonne de résine avec de l'eau , à stopper le rinçage de la colonne quand le taux de matière sèche dans le liquide de rinçage présente une valeur très faible, à éluer les polyphénols du type proanthocyanidines fixés sur la colonne de résine avec de l'éthanol aqueux, à recueillir l'éluat éthanolique que l'on soumet à l'évaporation sous vide en ajoutant régulièrement de l'eau au cours de l'évaporation pour obtenir un jus concentré, de sorte qu'en fin d'évaporation de l'alcool les polyphénols du type proanthocyanidines se trouvent dans un milieu purement aqueux, à évaporer la phase aqueuse par distillation sous vide poussé et à obtenir finalement le concentré de polyphénols du type proanthocyanidines sous une forme pulvérulente, contenant au moins 20 % de proanthocyanidines totales. De préférence, la fixation du jus concentré de Cranberry sur la résine s'effectue à contre-courant.

L'éthanol élue progressivement les polyphénols sans les altérer et la récupération de l'éluat est poursuivie jusqu'à disparition de la coloration violette dans l'éluat. On effectue de préférence un dosage des polyphénols dans l'éluat pour s'assurer que la totalité des polyphénols a bien été éluée.

Le débit est maintenu à 2BV/heure. Le volume d'éthanol aqueux, généralement nécessaire pour éluer la totalité des polyphénols fixés sur la résine est de 2BV. Selon une modalité particulière du procédé, le rinçage de la colonne après fixation des polyphénols s'effectue avec de l'eau, de préférence avec de l'eau pure, pour éliminer les composés non retenus par la résine. Le rinçage est effectué par le haut de la colonne, préférentiellement à un débit de 2BV/heure (BV=Bed Volume). Le rinçage de la colonne de résine est stoppé quand le taux de matière sèche de l'éluat est inférieur à 1 %. On a avantage à utiliser en général 2BV d'eau pour obtenir un rinçage efficace.

Selon un autre mode de réalisation préféré de l'invention, l'éluant hydroalcoolique est de préférence de l'éthanol aqueux à 96 %.

L'élution se fait par le haut de la colonne. L'éthanol décroche progressivement les polyphénols et la récupération de l'éluat est poursuivie jusqu'à disparition de la coloration violette dans l'éluat. Le débit est maintenu de préférence à 2BV/heure. Le volume nécessaire en général pour éluer la totalité des polyphénols de type proanthocyanidines fixés sur la résine est de l'ordre de 2BV.

Selon un autre mode de réalisation actuellement préféré, l'éluant alcoolique est récupéré et concentré par évaporation sous vide. De l'eau est régulièrement ajoutée au fur et à mesure que l'éthanol distille. Au final, les proanthocyanidines se trouvent dans un milieu entièrement aqueux.

Selon un autre mode de réalisation de l'invention, le résidu d'évaporation de l'éluat est amené à siccité, séché sur rouleaux sous vide, par atomisation ou par lyophilisation ou encore par freeze-drying ou tout autre moyen de séchage, pour obtenir une poudre fine.

On recueille ainsi un concentré de polyphénols sous forme d'une poudre de coloration violette à noire, contenant à ce stade au moins 20 % de proanthocyanidines de préférence de 25 à 30 % de proanthocyanidines et en particulier environ 26 % de proanthocyanidines.

Le rendement de l'extraction se situe au-dessus de 80 %, de préférence entre 80 à 90 % et en particulier à 84 %.

Le produit pulvérulent ainsi obtenu est stocké dans un endroit sec, à une température variant de 10 à 25°C à l'abri de la lumière. Un autre objet de l'invention porte sur l'utilisation des poudres de proanthocyanidines obtenues par un procédé tel que décrit précédemment pour la réalisation de compositions alimentaires ou diététiques à effet bénéfique pour la santé.

Le dosage des proanthocyanidines totales est réalisé par spectro photomètrie à l'aide du réactif vanilline.-acide sulfurique. Cette méthode très classique est décrite en détail dans M Pesez et J. Bartos « Principes de l'analyse colorimétrique ».

Selon un autre mode de réalisation de l'invention, le résidu d'évaporation est amené à siccité, séché sur rouleaux sous vide, par atomisation ou par lyophilisation ou encore par freeze-drying ou tout autre moyen de séchage.

On recueille ainsi un concentré de polyphénols sous forme d'une poudre de coloration violette à noire, contenant à ce stade au moins 20 % de proanthocyanidines de préférence de 25 à 30 % de proanthocyanidines et en particulier environ 26 % de proanthocyanidines.

Le rendement de l'extraction se situe au-dessus de 80 %, de préférence entre 80 à 90 % et en particulier à 84 %.

Le produit pulvérulent ainsi obtenu est stocké dans un endroit sec, à une température variant de 10 à 25°C à l'abri de la lumière.

Le dosage des proanthocyanidines totales est réalisé par spectro photomètrie à l'aide du réactif vanilline.-acide sulfurique. Cette méthode très classique est décrite en détail dans M Pesez et J. Bartos « Principes de l'analyse colorimétrique ».

Les exemples suivants fournissent les modes opératoires détaillés

### Exemple I

Le produit destiné à être fixé sur une colonne de résine correspond à du jus concentré de Cranberry (Vaccinum macrocarpon) contenant environ 5 % de matière sèche (50 ° Brix). Le produit est alors congelé (-18°C) et conditionné dans des fûts en acier de 200 1. Le produit ainsi stocké est de couleur pourpre à violet, légèrement visqueux, acide et sucré.

Le taux de proanthocyanidines sur ce produit frais et de 1,2 à 2,3 % environ sur sec selon la variété, la provenance ou le mode de culture de la plante.

### Préparation de la colonne de résine

Le jus de Cranberry est décongelé à température ambiante juste avant l'étape de dilution. On constate qu'un tel jus de Cranberry possède un taux de matière sèche (MS) de 50 % et que la majorité du résidu sec est constitué par des glucides (50° Brix).

Afin que le jus décongelé présente une fluidité satisfaisante dans la colonne, on ajuste le taux de matière sèche entre 20 et 25 %. On évite ainsi de cette façon l'encrassement prématuré des résines. L'ajustement du taux de matière sèche s'effectue par addition d'eau de ville à une température variant de 3 à 40°C au pH spontané de l'eau. Ce pH peut éventuellement être ajusté entre 2,5 et 8.

La colonne de résine utilisée est chargée avec une résine du type copolymère de divinyl benzène, commercialisée par la firme ROHM et HAAS sous la dénomination commerciale AMBERLITE XAD 16 HP.

### Fixation des polyphénols sur la résine

On effectue tout d'abord une mesure du volume apparent de résine (BV=Bed Volume) qui est important pour la suite des manipulations. Une fois la colonne chargée de résine, on y introduit un BV (Bed Volume) de jus préalablement dilué de Cranberry présentant de 20 à 25 % de matière sèche (MS), par exemple 500 litres de jus pour un volume de 500 litres de résine. De préférence, le volume de résine est introduit par le bas de la colonne, à contre courant, en éliminant l'eau dans laquelle la résine est en suspension, par la partie supérieure. La vitesse de chargement du jus dilué de Canneberge est d'environ 2BV par heure. La fraction non fixée qui s'écoule par la colonne, directement éluée, est récupérée.

Après introduction du volume total de jus, on effectue un rinçage de la colonne avec de l'eau. L'eau a pour effet d'éluer les composés non fixés par la résine, c'est à dire les sucres et les autres substances non polyphénoliques. Le rinçage est effectué par le haut de la colonne à un débit de 2BV par heure.

On stoppe le rinçage de la colonne quand le taux de matière sèche dans le liquide de rinçage est inférieur à 1 %. En général, le volume d'eau pour le rinçage est de 3BV pour obtenir un rinçage efficace.

Après l'étape de rinçage, on procède à l'étape de récupération des polyphénols adsorbés. Cette étape est effectuée en éluant les composés adsorbés sur la résine avec de l'éthanol à 96 %. L'élution s'effectue par le haut de la colonne et le liquide s'écoule par le bas. L'éthanol provoque un décrochement progressif de la coloration violette-signe de la présence de proanthocyanidines dans l'éluat.

Il est recommandé de procéder lors de cette étape, au dosage des polyphénols pour s'assurer que la totalité des polyphénols a bien été éluée.

Le débit est maintenu à 2BV/heure. Le volume d'éthanol à 96°C nécessaire en général pour éluer la totalité des polyphénols fixés sur la résine est de 2BV.

### Concentration

Après que la totalité de l'éluat éthanolique a été rassemblée, on concentre celui-ci par évaporation sous vide en ajoutant régulièrement de l'eau dans le liquide lors de l'évaporation de l'éthanol de manière à ne pas risquer de détériorer ou de décomposer les polyphénols. A la fin de l'évaporation de l'éthanol, les proanthocyanidines se trouvent dans un milieu à 100 % aqueux.

On procède ensuite à l'évaporation de l'eau par distillation sous vide poussé et on obtient finalement une poudre fine, de couleur violette à noire, qui contient à ce stade au moins 20 % de proanthocyanidines totales.

La poudre est stockée dans un endroit sec à une température variant de 10 à 25°C à l'abri de la lumière.

Le taux de récupération des proanthocyanidines par cette méthode est d'au moins 80 %.

### Exemple II

### Extraction des proanthocyanidines

On garnit une colonne en acier inox de 2000 l avec un volume de résine Amberlite XAD 16 HP de 500 l. On y introduit 500 l de jus de Cranberry titrant 20 à 25 % de matière sèche. Le débit de chargement sur la colonne est de 1000 l par heure.

Les colonnes sont ensuite rincées à l'aide de 1500 l d'eau versée par le haut de la colonne. Le débit de rinçage est d'environ 1000 l par heure.

On procède après ce lavage à l'élution des polyphénols de la colonne par addition de 1000 l d'éthanol aqueux. Le débit utilisé reste inchangé à savoir 2BV/heure.

L'éthanol de l'éluat est progressivement évaporé sous pression réduite tout en ajoutant de 50 à 100 l d'eau dans le réacteur afin de laisser les polyphénols en solution lorsque la totalité de l'éthanol est évaporée.

Après évaporation complète de l'éthanol, la solution aqueuse restante est extraite du réacteur puis évaporée à sec sur rouleaux sous vide, et ensuite séchée par atomisation ou par lyophilisation pour obtenir une poudre cristalline violette à noire riche en proanthocyanidines. Ces poudres trouvent une utilisation en alimentation et/ou en diététique pour réaliser des compositions ou des aliments ayant des propriétés anti-oxydantes.

### Exemple III

### Supplément alimentaire

| | | | |
|---|---|---|---|
| ▪ | Poudre de concentré polyphénolique de Vaccinium macrocarpon | 0,100 kg | |
| ▪ | Caséinate de calcium | 0,250 | kg |
| ▪ | Mannitol | 0,025 | kg |
| ▪ | Aspartame | 0,00025 | kg |
| ▪ | Lait en poudre dégraissé | 0,175 | kg |

pour 1000 sachets de poudre

### Exemple IV

### Biscuits à base d'extrait de Vaccinium

| | | | |
|---|---|---|---|
| ▪ | Poudre de concentré polyphénolique de Vaccinium macrocarpon | 0,025 | kg |
| ▪ | Eau | 0,150 | kg |
| ▪ | Lécithine d'oeuf | 0,050 | kg |
| ▪ | Sucre | 0,060 | kg |
| ▪ | Farine de blé | 0,341 | kg |
| ▪ | Emulsifiant E 4726 (2,01 %) | 0,02 | kg |
| ▪ | Cellulose microcristalline | 0,030 | kg |
| ▪ | Poudre levante | 0,04 | kg |

### Biscuits à base de concentré de Vaccinium

| | | | |
|---|---|---|---|
| ▪ | Poudre d'extrait de Vaccinium macrocarpon | 0,200 | kg |
| ▪ | Farine de blé | 0,275 | kg |
| ▪ | Sucre cristal | 0,350 | kg |
| ▪ | Eau | 0,200 | kg |
| ▪ | Lécithine de soja | 0,050 | kg |
| ▪ | Dinalplus | 0,600 | kg |
| ▪ | Emulsifiant E 4726 (0,85 %) | 0,01 | kg |
| ▪ | Poudre levante E450 ai-E500 ii (0,85 %) Dinal plus = protéines lactiques, protéines végétales | qs | |

(farine de pois, farine de riz, fécule de pomme de terre)
pour 1000 biscuits d'un poids moyen de 1,50 g

### Capsules molles

| | | | |
|---|---|---|---|
| ▪ | Phospholipides cérébraux | 0,016667 | kg |
| ▪ | Extrait concentré de Vaccinium macrocarpon | 0,700 | kg |
| ▪ | Oléorésine de carottes | 0,100 | kg |
| ▪ | Concentré d'huile végétale riche en tocophérol | 0,005 | kg |

pour 1000 capsules finies au poids de 0,800 g

## Revendications

1. Procédé d'extraction des polyphénols du type proanthocyanidines du jus de Cranberry (Vaccinium macrocarpon) qui consiste :
- à éliminer en premier lieu les substances inertes contenues dans ce jus par dilution à l'eau du jus concentré,
- à fixer sur un matériau adsorbant polymère du type styrène/divinyl benzène, les polyphénols du type proanthocyanidines,
- à rincer la colonne de résine avec de l'eau,
- à stopper le rinçage de la colonne quand le taux de matière sèche dans le liquide de rinçage présente une valeur très faible,
- à éluer les polyphénols du type proanthocyanidines fixés sur la colonne de résine avec de l'éthanol aqueux,
- à recueillir l'éluat éthanolique que l'on soumet à l'évaporation sous vide en ajoutant régulièrement de l'eau au cours de l'évaporation pour obtenir un jus concentré, de sorte qu'en fin d'évaporation de l'alcool les polyphénols du type proanthocyanidines se trouvent dans un milieu purement aqueux,
- à évaporer la phase aqueuse par distillation sous vide poussé et
- à obtenir finalement le concentré de polyphénols du type proanthocyanidines sous une forme pulvérulente, contenant au moins 20 % de proanthocyanidines totales.

2. Procédé selon la revendication 1 dans lequel la fixation du jus concentré de Cranberry sur la résine s'effectue à contre-courant.

3. Procédé selon la revendication 1 dans lequel le rinçage de la colonne de résine après fixation, s'effectue avec de l'eau pure.

4. Procédé selon la revendication 1 dans lequel le rinçage de la colonne de résine après fixation des polyphénols du type proanthocyanidines s'effectue à un débit de 2BV/heure (BV=Bed Volume).

5. Procédé selon la revendication 1 dans lequel le liquide d'élution de la résine après fixation est de l'éthanol aqueux à 96%.

6. Procédé selon la revendication 1 dans lequel le débit d'éthanol aqueux pour l'élution de la colonne de résine est de 2BV/heure.

7. Procédé selon la revendication 1 dans lequel le volume nécessaire pour éluer la totalité des polyphénols du type proanthocyanidines fixés sur la résine est de l'ordre de 2BV.

8. Procédé selon la revendication 1 dans lequel le résidu d'évaporation de l'éluat est amené à siccité, séché sur rouleaux sous vide, par atomisation ou par lyophilisation ou encore par freeze-drying ou tout autre moyen de séchage, pour obtenir une poudre fine titrant 26% environ de proanthocyanidines.

9. Procédé selon la revendication 1 dans lequel le rendement d'extraction des proanthocyanidines varie de 80 à 90%.

10. Utilisation des poudres de proanthocyanidines obtenues par le procédé selon les revendications précédentes pour la réalisation de compositions alimentaires ou diététiques à effet bénéfique pour la santé.

## Patentansprüche

1. Verfahren zum Extrahieren der Polyphenole vom Typ Proanthocyanidine des Cranberrysafts (Vaccinium macrocarpon), das darin besteht:
- zunächst die inerten Stoffe, die in diesem Saft enthalten sind, durch Verdünnen des konzentrierten Safts mit Wasser zu beseitigen,
- die Polyphenole vom Typ Proanthocyanidine an ein adsorbierendes Polymermaterial vom Typ Styrol/Divinylbenzol zu fixieren,
- die Harzsäule mit Wasser zu spülen,
- das Spülen der Säule zu stoppen, wenn der Gehalt an Trockensubstanz in der Spülflüssigkeit einen sehr niedrigen Wert aufweist,
- die Polyphenole vom Typ Proanthocyanidine, die an der Harzsäule fixiert sind, mit wässrigem Ethanol zu eluieren,
- das ethanolische Eluat aufzufangen, das, um einen konzentrierten Saft zu erhalten, der Vakuumverdampfung unter regelmäßigem Zugeben von Wasser während des Verdampfens unterzogen wird, sodass sich die Polyphenole vom Typ Proanthocyanidine am Ende des Verdampfens des Alkohols in einem rein wässrigen Medium befinden,
- die wässrige Phase durch Hochvakuumdestillation zu verdampfen, und
- schließlich das Konzentrat von Polyphenolen vom Typ Proanthocyanidine in einer Pulverform zu erhalten, die mindestens 20 % Gesamt-Proanthocyanidine enthält.

2. Verfahren nach Anspruch 1, wobei das Fixieren des konzentrierten Cranberrysafts am Harz im Gegenstrom erfolgt.

3. Verfahren nach Anspruch 1, wobei das Spülen der Harzsäule nach dem Fixieren mit reinem Wasser erfolgt.

4. Verfahren nach Anspruch 1, wobei das Spülen der Harzsäule nach dem Fixieren der Polyphenole vom Typ Proanthocyanidine mit einem Durchsatz von 2 BV/Stunde (BV = Bed Volume) erfolgt.

5. Verfahren nach Anspruch 1, wobei die Elutionsflüssigkeit des Harzes nach dem Fixieren 96 %-iges wässriges Ethanol ist.

6. Verfahren nach Anspruch 1, wobei der Durchsatz an wässrigem Ethanol zum Eluieren der Harzsäule 2 BV/Stunde beträgt.

7. Verfahren nach Anspruch 1, wobei das Volumen, das erforderlich ist, um die Gesamtheit der am Harz fixierten Polyphenole vom Typ Proanthocyanidine zu eluieren, in der Größenordnung von 2 BV liegt.

8. Verfahren nach Anspruch 1, wobei der Verdampfungsrückstand des Eluats zur Trockne gebracht, auf Walzen vakuumgetrocknet, durch Zerstäuben oder durch Lyophilisieren oder auch durch Gefriertrocknen oder jedes andere Trocknungsmittel getrocknet wird, um ein feines Pulver mit einem Titer von etwa 26 % Proanthocyanidine zu erhalten.

9. Verfahren nach Anspruch 1, wobei die Extraktionsausbeute der Proanthocyanidine von 80 bis 90 % variiert.

10. Verwendung der über das Verfahren nach den vorstehenden Ansprüchen erhaltenen Proanthocyanidin-Pulver zur Herstellung von Nahrungsmittel- oder diätischen Zusammensetzungen mit günstiger Wirkung für die Gesundheit.

## Claims

1. A method for extracting proanthocyanidin-type polyphenols from Cranberry juice (Vaccinium macrocarpon) which consists in:
- first of all, eliminating the inert substances contained in this juice by dilution of the concentrated juice with water,
- fixing the proanthocyanidin-type polyphenols on a polymeric adsorbent material of the styrene / divinyl benzene type,
- rinsing the resin column with water,
- stopping the rinsing of the column when the dry matter content in the rinsing liquid has a very low value,
- eluting the proanthocyanidin-type polyphenols fixed on the resin column with aqueous ethanol,
- collecting the ethanolic eluate which is subjected to vacuum evaporation by regularly adding water during the evaporation to obtain a concentrated juice, so that at the end of evaporation of the alcohol the proanthocyanidin-type polyphenols are in a purely aqueous medium,
- evaporating the aqueous phase by high vacuum distillation and
- finally obtaining the proanthocyanidin-type polyphenols concentrate in a pulverulent form, containing at least 20% of total proanthocyanidins.

2. The method according to claim 1, wherein the fixation of the concentrated Cranberry juice on the resin is performed in countercurrent.

3. The method according to claim 1, wherein the rinsing of the resin column after fixation is performed with pure water.

4. The method according to claim 1, wherein the rinsing of the resin column after fixation of the proanthocyanidin-type polyphenols is performed at a flow rate of 2BV / hour (BV = Bed Volume).

5. The method according to claim 1, wherein the elution liquid of the resin after fixation is 96% aqueous ethanol.

6. The method according to claim 1, wherein the aqueous ethanol flow rate for the elution of the resin column is 2BV / hour.

7. The method according to claim 1, wherein the volume required to elute all the proanthocyanidin-type polyphenols fixed on the resin is in the range of 2BV.

8. The method according to claim 1, wherein the residue of the eluate evaporation is brought to dryness, dried on rollers under vacuum, by atomization or by lyophilization or else by freeze-drying or any other drying means, in order to obtain a fine powder containing about 26% of proanthocyanidins.

9. The method according to claim 1, wherein the extraction yield of proanthocyanidins ranges from 80 to 90%.

10. A use of the proanthocyanidin powders obtained by the method according to preceding claims for producing food or dietetic compositions with a beneficial effect on health.
